# EUROPEAN PATENT SPECIFICATION

(11) **EP 2 502 582 B1**
(45) Date of publication and mention of the grant of the patent: **30.11.2016**
(21) Application number: 12161094.3
(22) Date of filing: 23.03.2012
(51) Int. Cl.: A61B 17/17, A61B 17/16

(54) **Guiding assembly for spinal drilling operation**
Führungseinrichtung für eine Bohroperation der Wirbelsäule
Ensemble de guidage pour une opération de forage de la colonne vertébrale

(30) Priority: 25.03.2011 TW 100110288; 13.03.2012 TW 101108536
(43) Date of publication of application: 26.09.2012
(73) Proprietor: National Cheng Kung University, Tainan City (TW)
(72) Inventor: Fang, Jing-Jing, 701 East Dist., Tainan City (TW); Lin, Ruey-Mo, 701 Tainan City (TW); Wu, Tzu-Chieh, 730 Xinying Dist. , Tainan City (TW); Hou, Cheng-Yu, 112 Beitou Dist. Taipei City (TW)
(74) Representative: 2K Patentanwälte Blasberg Kewitz & Reichel

(56) References cited:
- US-A- 4 696 308
- US-A1- 2008 077 152
- US-A1- 2008 183 297
- US-A1- 2010 023 018
- US-A1- 2010 152 793

## Description

### BACKGROUND OF THE INVENTION

### Field of Invention

The present invention relates to a guiding assembly for drilling operation, and in particular, to a guiding assembly for spinal drilling operation, wherein the methods, not claimed, represent background art that is useful for better understanding the invention.

### Related Art

Spine is such an important element in human body because it not only constructs the main frame of skeleton but has the function of protecting the inside spinal cord, which is the spindle of human nerve system. The nerves protrude outwardly from the inside of the vertebrae, and then extend forwardly, laterally, and vertically to the visceral organs as well as the extremity of human body. In other words, the vertebrae are the origin point of the neural network system of human body.

Vertebrae include cervical vertebrae, thoracic vertebrae, and lumbar vertebrae. Nerves come from the spinal cord and go out through the intervals between the vertebral bodies. Unfortunately, if the deformation of any vertebral body of the vertebrae occurs, the nerve is possibly pressed, which may indirectly affect the organ, muscle and gland body connected to the nerve. This undesired deformation can result a huge injury to human health.

The reasons of the vertebral dislocation include the spondylolisthesis caused by vertebral degeneration, injury (e.g. dislocation or fracture), infection, tumer, and the likes. The major therapy for the vertebrae deformation is to implant a transpedicular screw on the pedicle of a vertebra (or vertebral body). The rigidity of the transpedicular screw can push the vertebra back the normal position and fix it, so that the relative positions of the vertebrae are more stable so as to avoid the compression and wearing of nerves and release the pain of patient.

In the recent years, the spinal surgery has been progressively developed. Especially, the transpedicular screw further provides a revolutionary progress, and it can be applied to not only the easier lumbar vertebrae operation but also the more difficult cervical and thoracic vertebrae operation. However, the implantation of transpedicular screw still exists a certain possibility of complication, especially for the cervical and thoracic vertebrae operation. This unpredictable risk of these surgeries really stops many patients.

Since the shape of each vertebra may different in different bodies, it has been an important issue of the surgery to precisely implant the transpedicular screw into the center of the pedicle of the targeted vertebra.

Accordingly, the location methods applied in the spinal drilling operation have been studied. The conventional location methods are mainly divided three types. The first type includes the steps of preoperation CT scan and intraoperative registration. The second type is to use an adjustable operative accessory for measuring before the surgery and then perform the operation with the well-adjusted operative accessory. The third type is to produce a customized guiding element for a specific patient to perform the operation.

However, although the first type can achieve a good precision, it spends a lot time on registration. Besides, the operator must confirm the angles and positions again and again during the operation. These complex and extra procedures can interfere with the operation. Regarding to the second type, the precise guiding result is merely achieved because the errors can be caused by manual operation and, more important, the position of the target vertebra may different before and during the surgery.

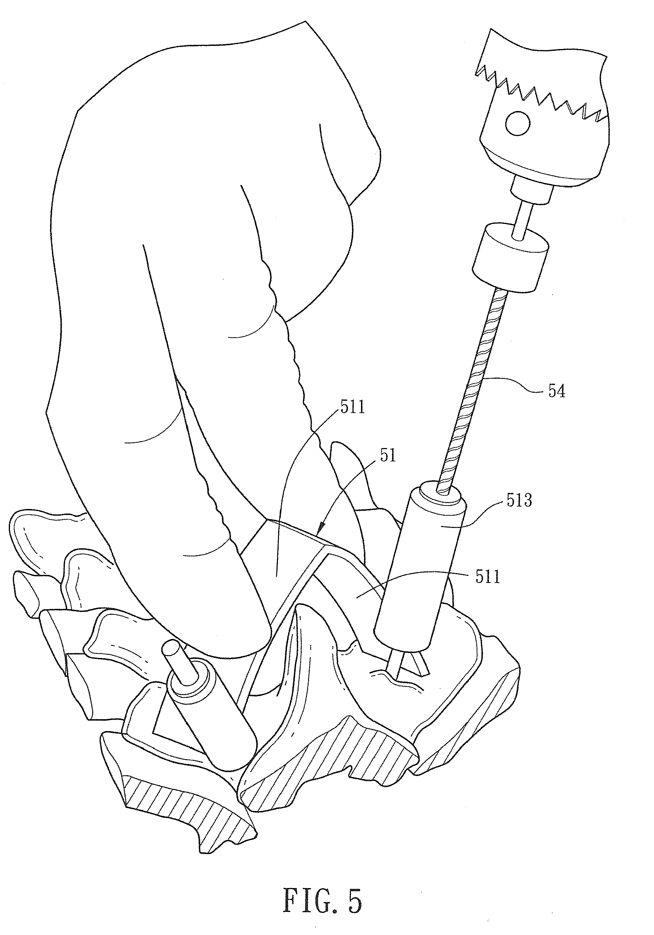

The third method need more time and cost on the preparation for customization design before the surgery, but it has the advantages of high precision and easy operation. As shown in FIG. 5, the convention method is to utilize an auxiliary stand 51 during the operation with the customized guiding element 54. The auxiliary stand 51 includes two support legs 511, each of which is configured with a guide 513. The auxiliary stand 51 is designed with a V-shaped knife-edge for standing on the spinous process and transverse process of the vertebra. During the operation, the operator places the auxiliary stand 51 to contact against the spinous process and transverse process of the vertebra so as to locate the auxiliary stand 51, and then uses the guide 513 to guide the drill 514 for successfully performing the reaming procedure on the vertebra.

Although the above method can reduce the error in the operation, it has a problem in keeping the auxiliary stand at the desired position. In details, the auxiliary stand is stood on the surface of the vertebra through the bottom of the support legs only, and the body of the auxiliary stand does not configured with any other mechanism for contacting or fastening to the vertebra. Thus, the auxiliary stand is easily swayed even the operator carefully holds it. In addition, the guide of the auxiliary stand can provide the reference for the drilling location, but it may lose stability during the drilling/reaming, and even worse, the auxiliary stand may misalign with the center of the pedicle. Thus, the location stability during the reaming procedure is still insufficient.

Therefore, it is an important subject of the present invention to provide a guiding tool for spinal drilling operation that is easily operated and has the specificity for patient's vertebra and high location stability and precision, thereby improving the efficiency and application of the customized guiding element on the location and drilling/reaming issues during spinal drilling operation.
US2010023018 A describes a bilateral drilling and screw placement guide adapted for fixation to the spinous process of a vertebral body. The positioning and surgical guiding instrument is adapted for use during a spinal surgical procedure in conjunction with a drilling tool, fastening device, e.g. a pedicle screw, K-wire or the like. The guide includes an engagement device for attachment to a region of the spinous process, to which an adjustably attached support member is affixed. This allows for immobilizing the guide upon the spinous process anatomical landmark, in a specific orientation with respect thereto. The guide includes a bilaterally adjustable drill guide assembly for precise anatomical positioning of the drill and screw placement guides bilaterally about the sagittal, axial and coronal planes so as to enable the defining of a plurality of drilling axes extending toward the vertebral body. The device further provides for adjustment to account for anatomical variations in width along the axial plane, and includes pointing and angular positioning functionality to insure repeatable and reliable pilot hole and screw placement along a plurality of angles.
US4696308 A describes an apparatus for obtaining a core sample which includes an elongate guide pin and a hollow drill element. The drill element has an open front end provided with a cutting edge and an open rear end provided with an axial engagement structure for engaging an associated driving tool. A longitudinal aperture extends from the front end of the drill element to the rear end with the aperture having a smooth uninterrupted interior periphery throughout at least a major portion of its length. A spacer slug is adapted to be positioned in the drill element longitudinal aperture. The spacer slug has a centrally disposed aperture extending longitudinally therethrough and sized so that the guide pin can extend therethrough. The spacer slug has a suitable outer diameter so that it can slide in the drill element longitudinal aperture.
US2010152793 A describes a system and method for making an access channel through a vertebral body to access a site of neural compression, decompressing it, and repairing the channel to restore vertebral integrity. System elements include an implantable vertebral plate, a guidance device for orienting bone cutting tools and controlling the path of a cutting tool, a bone cutting tool to make a channel in the vertebral body, a tool for opening or partially-resecting the posterior longitudinal ligament of the spine, a tool for retrieving a herniated disc, an implantable device with osteogenic material to fill the access channel, and a retention device that lockably-engages the bone plate to retain it in position after insertion. System elements may be included in a surgery to decompress an individual nerve root, the spinal cord, or the cauda equina when compressed, for example, by any of a herniated disc, an osteophyte, a thickened ligament arising from degenerative changes within the spine, a hematoma, or a tumor.
US2008183297 A describes a method and set of surgical instruments for fitting a shoulder prosthesis, and the shoulder prosthesis. The proposed method seeks to interpose a bone graft between the previously prepared glenoid surface (G) of a scapula (S) of a patient's shoulder and the face of a glenoid prosthetic component opposite the articular surface. The set of instruments permit the bone graft to be taken from the upper epiphysis of the humerus (H), either in situ or ex vivo.
US 2011/0319745 describes a system and method for developing customized apparatus for use in one or more surgical procedures. The system and method incorporates a patient's unique anatomical features or morphology, which may be derived from capturing MRI data or CT data, to fabricate at least one custom apparatus. According to a preferred embodiment, the customized apparatus comprises a plurality of complementary surfaces based on a plurality of data points from the MRI or CT data. Thus, each apparatus may be matched in duplicate and oriented around the patient's own anatomy, and may further provide any desired axial alignments or insertional trajectories. In an alternate embodiment, the apparatus may further be aligned with at least one other apparatus used during the surgical procedure.
US 4 907 577 describes a spinal transpedicle drill jig adapted for providing a safe route for drilling, including an I-shaped body, a guiding base and a positioning base. The jig provides a precise location for drilling to prevent deviation of the drilling direction so as to prevent injury during surgery to the nerve root or spinal cord.
US 2008/0021480 describes a system for attaching first and second fixation elements at a predetermined angle relative to each other to first and second locations of a vertebra, respectively includes a first guide wire configured to be inserted into the first location of the vertebra, an angular guide system comprising first and second guide arms configured to be set at the predetermined angle relative to each other and the first guide arm is configured to be inserted over the first guide wire and a second guide wire configured to be inserted through the second guide arm into the second location of the vertebra. The second guide wire includes first and second members configured to pivot relative to each other.
"NOVEL PATIENT-SPECIFIC NAVIGATIONAL TEMPLATE FOR CERVICAL PEDICLE SCREW PLACEMENT" (Sheng Lu; etc; SPINE, 20091215 Lippincott Williams & Wilkins, US, Vol:34, Nr:26, Pages:E959 - E964) describes a navigational template for cervical pedicle screw placement. The accuracy of the navigational template was examined by visual inspection, RP model of vertebra and navigational template.
"A novel computer-assisted drill guide template for lumbar pedicle screw placement: a cadaveric and clinical study (Sheng Lu; etc; The International Journal of Medical Robotics and Computer Assisted Surgery, 20090601, Vol:5, Nr:2, Pages:184 - 191)" describes a drill template which was created using UG Imageware 12.1, with a surface that is the inverse of the vertebral surface. The drill template and its corresponding vertebra were manufactured using RP. The accuracy of the drill template was confirmed by preoperatively drilling the screw trajectory into the vertebra biomodel.

### SUMMARY OF THE INVENTION

The present invention relates to a guiding assembly as claimed hereafter Preferred embodiments of the invention are set forth in the dependent claims. In view of the foregoing subject, an objective of the present invention is to provide a guiding assembly for spinal drilling operation that includes a guiding element, an auxiliary element, a k-pin and a cannular driller. The guiding assembly is easily operated and has the specificity for patient's vertebra and high location stability and precision, so that it can improve the efficiency and application of the customized guiding element on the location and drilling/reaming issues during spinal drilling operation.

Another objective of the present disclosure is to provide a method, not claimed, for spinal drilling operation, the method represents background art that is useful for better understanding the invention, the method is performed for operating the guiding element, auxiliary element, k-pin and cannular driller on set purpose and step by step so as to properly use the specific functions and the auxiliary properties of the elements, so that the operator can simply and rapidly perform the drilling operation and still keep the desired high location stability and precision.

To achieve the above objectives, the disclosure discloses a, not claimed, for spinal drilling operation, the method represents background art that is useful for better understanding the invention, the method including the following steps of: disposing a guiding element on a vertebra, wherein the guiding element comprises a main body, at least one connection part, and at least one locating part, the main body has at least one stand portion and at least one contact portion connecting with the stand portion, and the locating part connects to the stand portion through the connection part; mounting an auxiliary element on the locating part; mounting a k-pin on the auxiliary element; locating the k-pin and removing the auxiliary element from the locating part; mounting a cannular driller having a holding part and a drilling part through the k-pin; and rotating the holding part to drive the drilling part for a reaming process.

In one embodiment, the main body further has a hand-held portion connecting to the stand portion. Preferably, the hand-held portion has a through hole.

In one embodiment, the main body further has at least one fixing portion connecting to the contact portion.

In one embodiment, the main body is disposed at a transverse process of the vertebra, and one side of the contact portion in contact against the vertebra has a curvature corresponding to the surface of the transverse process.

In one embodiment, the main body has two stand portions, and the main body is disposed across a spinous process of the vertebra through the stand portions.

In one embodiment, the main body has two contact portions connecting to the stand portions, respectively, and one side of the contact portion in contact against the vertebra has a curvature corresponding to the surface of the spinous process.

In one embodiment, the guiding element has two connection parts, and the connection parts are connected to the same side of the stand portion and form an angle. Preferably, the connection part and the stand portion form an angle at one side of the vertebra, and the angle is large than 90 degrees.

In one embodiment, the auxiliary element has a through hole along a longitudinal direction, and the k-pin passes through the through hole in the step of mounting the k-pin on the auxiliary element.

In one embodiment, the cannular driller is a T-shaped driller.

In addition, the present invention further discloses a guiding assembly for spinal drilling operation, which includes a guiding element, an auxiliary element, a k-pin, and a cannular driller. The guiding element is configured for disposing on a vertebra. The guiding element includes a main body having two stand portions and two contact portions respectively connecting with the stand portions, two connection parts, and two locating parts connecting to the stand portions of the main body through the connection parts, respectively. The auxiliary element is mounted on one of the locating parts, and the k-pin is mounted on the auxiliary element. The cannular driller is mounted on the k-pin and has a holding part and a drilling part. The main body is disposed across a spinous process of the vertebra through the stand portions, and the main body in contact against surface of a lamina of the vertebra.

In one embodiment, the main body further has a hand-held portion connecting to the stand portions. Preferably, the hand-held portion has a through hole.

In one embodiment, the main body further has two fixing portions connecting to the contact portions, respectively.

In one embodiment, one side of each of the fixing portions in contact against the vertebra has a curvature corresponding to the surface edge of the lamina.

In one embodiment, one side of each of the contact portions in contact against the vertebra has a curvature corresponding to the surface of the lamina.

In one embodiment, the connection parts are connected to the same side of the stand portion and form an angle. Preferably, the connection part and the stand portion form an angle at one side of the vertebra, and the angle is large than 90 degrees.

In one embodiment, the auxiliary element has a through hole along a longitudinal direction, and the k-pin passes through the through hole in the step of mounting the k-pin on the auxiliary element.

In one embodiment, the cannular driller is a T-shaped driller.

As mentioned above, the guiding assembly for spinal drilling operation of the present invention is composed of several elements, including the guiding element, auxiliary element, k-pin and cannular driller, so that the stability and precision of the reaming procedure during the spinal drilling operation can be enhanced.

Moreover, the specific functions of the above elements can help the spinal drilling operation. Regarding to the guiding element, since it has simple structure and is suitable for customization, the demand of the patient can be satisfied. Besides, the guiding element has a portion fitting the vertebra, so that the connection between the guiding element and the vertebra can be further improved. In addition, the auxiliary element and the cannular driller are configured cooperating with the locating part. The auxiliary element can assist the location of the k-pin and prevent the non-stability in the conventional hand-hold procedure. The cannular driller can perform the reaming process through the locating part, thereby increasing the precision and decreasing the safety of spinal drilling operation.

Compared with the conventional art, the present invention still remains the conventional advantages of customizable and easy operation, and can further provide higher location stability and precision due to the structural property of the guiding element. Furthermore, either the method of the guiding assembly for spinal drilling operation utilizes an auxiliary element for facilitating and adjusting the penetrating position of the k-pin. This configuration can precisely match the hole drilled by the cannular driller with the targeted pedicle, so that the surgeons can precisely drill into the center of the pedicle of the targeted vertebra.

Preferably, the guiding element of the present invention can have customized design based on the surface angle of the targeted vertebra (e.g. cervical, thoracic or lumber vertebra) of the patient. Accordingly, when the fixing portion is pressed so as to push the guiding element, the guiding element can be still fixed on the targeted vertebra firmly, thereby increasing the precision and safety of the spinal drilling operation.

### BRIEF DESCRIPTION OF THE DRAWINGS

The invention will become more fully understood from the detailed description and accompanying drawings, which are given for illustration only, and thus are not limitative of the present invention, and wherein:

FIGS. 1A to 1E are schematic diagrams showing different elements of a guiding assembly for spinal drilling, wherein FIGS. 1A to 1B representing background art that is useful for better understanding, and wherein FIGS. 1C to 1E represent different elements of a guiding assembly according to an embodiment of the present invention.

FIGS. 2A and 2B are schematic diagrams showing a guiding element of the guiding assembly for spinal. FIGS. 2A and 2B represent background and that is useful for better understanding the invention.

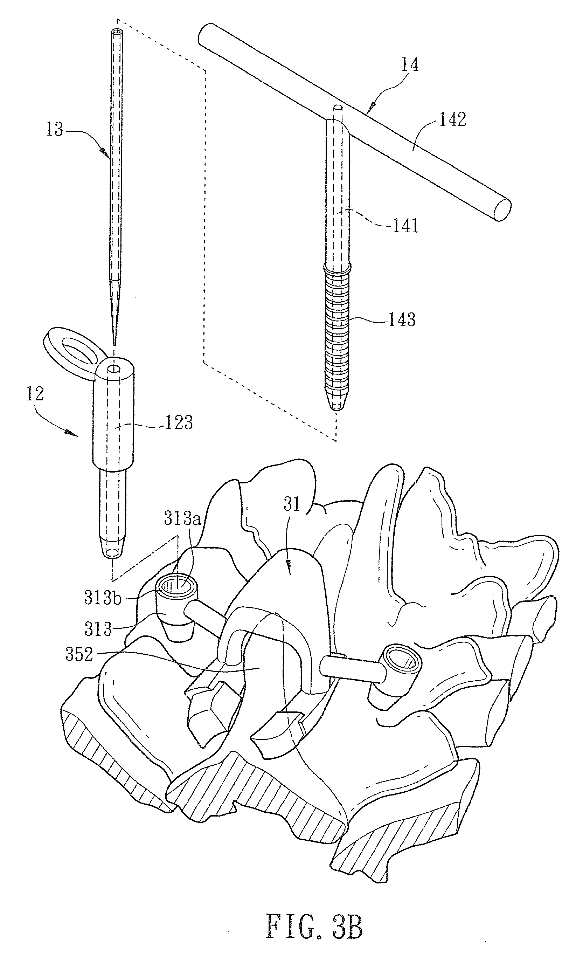

FIGS: 3A and 3B are schematic diagrams showing other guiding elements of the guiding assembly for spinal drilling operation, wherein FIS. 3A represents the embodiment of the invention and FIGS. 3B represents background art that is useful for better understanding the invention;

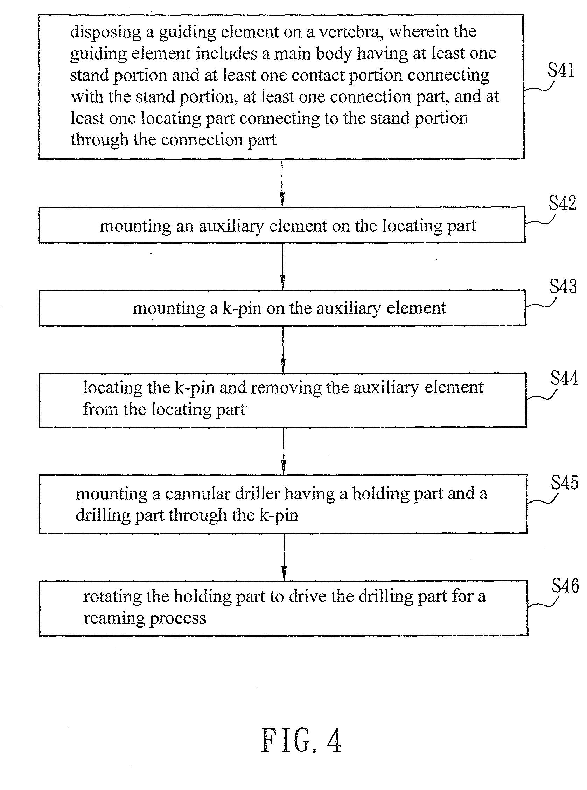

FIG. 4 is a flow chart of a method, not claimed, for spinal drilling operation according to the preferred embodiment of the present invention; and

FIG. 5 is a schematic diagram showing the conventional customized guiding element and auxiliary stand for spinal drilling operation.

### DETAILED DESCRIPTION OF THE INVENTION

The present invention will be apparent from the following detailed description, which proceeds with reference to the accompanying drawings, wherein the same references relate to the same elements.

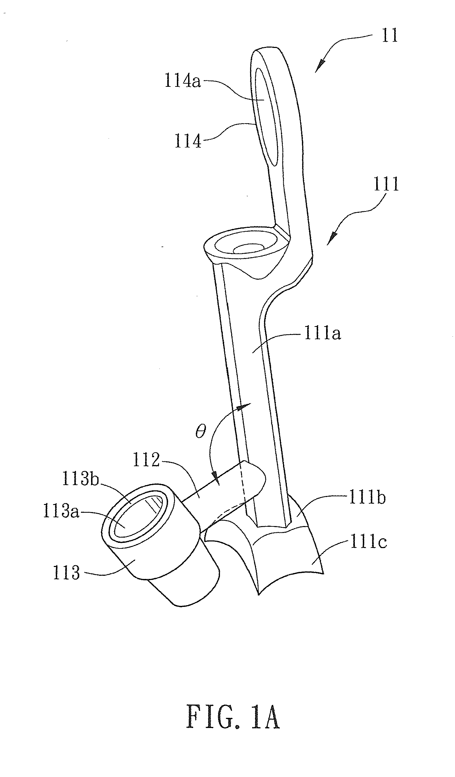

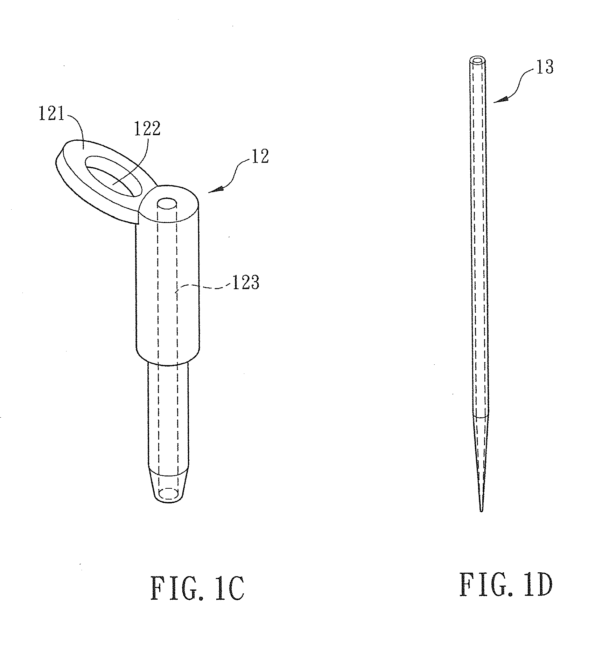

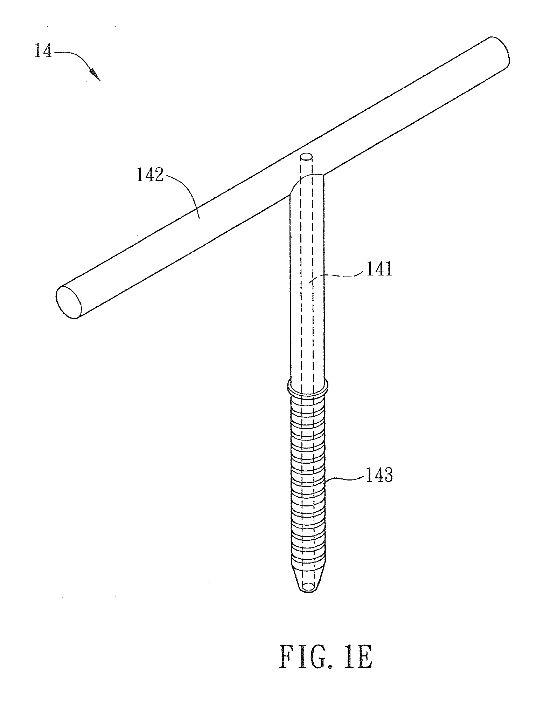

FIGS. 1A to 1E are schematic diagrams showing different elements of a guiding assembly for spinal drilling operation. Referring to FIGS. 1A to 1E, a guiding assembly for spinal drilling operation includes a guiding element 11, an auxiliary element 12, a k-pin 13, and a cannular driller 14. The structural features of these elements will be described hereinafter.

With reference to FIG. 1A, the guiding element 11 includes a main body 111, a connection part 112, and a locating part 113.

The main body 111 has at least one stand portion 111a and at least one contact portion 111b. The stand portion 111a is mostly a vertical column, and the bottom thereof connects to the contact portion 111b. The main body 111 further has at least one fixing portion 111c disposed at one end of the contact portion 111b along a longitudinal direction. In this embodiment, the fixing portion 111 c is configured at the front and back sides of the end of the contact portion 111b along the longitudinal direction.

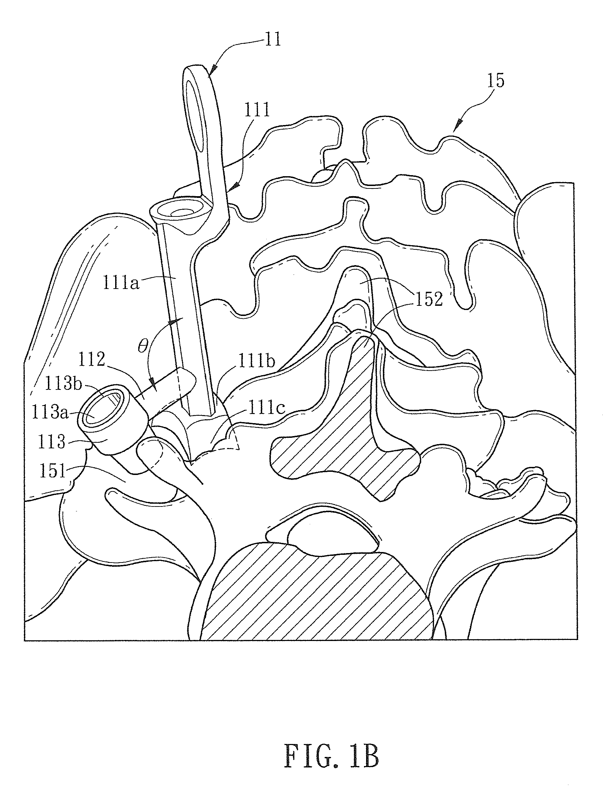

Referring to FIG. 1B, the main body 111 can be disposed on the transverse process 151 of the vertebra 15 by the supporting of the stand portion 111a and the contact of the contact portion 111b. Of course, the transverse process 151 of the vertebra 15 is only an exampled location of the guiding element 11, and in practice, the guiding element 11 may be alternatively disposed on the spinous process 152 of the vertebra 15 (refer to the following embodiment).

The fixing portion 111c has a hook shape, so that the contact portion 111b and the transverse process 151 can be further connected by this locking means. Besides, one side of the contact portion 111b in contact against the vertebra 15 has a curvature corresponding to the surface of the transverse process 151. Accordingly, the connection between the contact portion 111b and the vertebra 15 can be enhanced so as to prevent the possible swaying the guiding element 11 and increase the stability.

The locating part 113 connects to one side of the stand portion 111a through the connection part 112. Preferable, the locating part 113 connects to one side of the stand portion 111a, which is relatively located at the outer side of the spinous process 152. As shown in FIG. 1B, the connection part 112 and the stand portion 111a and the outer side of the vertebra 15 form an angle θ, which is preferably larger than, for example but not limited to, 90 degrees. In practice, the angle θ can be customized according to the shape of the vertebra 15 of the patient and the angle of the drill hole to be formed. If the angle is larger than 90 degrees, the connection part 112 may extend downwards obliquely so as to facilitate the reaming process.

The locating part 113 has a through hole 113a that is provided to confirm the drilling location. Since the guiding element 11 is manufactured based on the information of the vertebra 15 of the patient and the optimum drilling angle and direction estimated by expert or computer, the drilling location can be rapidly figured out by the through hole 113a of the locating part 113 according to the fixing and connecting relations and directions of all elements and parts when the guiding element 11 is provided on the vertebra 15. For example, the projection of the center of the through hole 113a on the vertebra 15 may indicate the center of the location of the drilled hole.

All elements of the above-mentioned guiding element 11 may be integrally formed so as to achieve high stability and precision of the guiding element 11. Of course, the elements of the above-mentioned guiding element 11 may also be individually formed and then assembled by adhering or locking.

In this embodiment, the guiding element 11 is mainly made of resin material such as ABS (SL7580, SL7565 or SL7520, and preferably SL7580). Of course, in other embodiments, the guiding element may be made of any other materials, which has high impact durability, high rigidity and no bio-toxicity, and is easily processed, so that the complexness of the customization of the guiding element can be minimized.

Besides, the guiding element 11 of the embodiment may further include a hand-held portion 114, which is disposed on one side of the stand portion 111a away from the contact portion 111b (upper end in this case). The configuration of the hand-held portion 114 helps the operator to easily hold and operate the guiding element 11. Preferably, the hand-held portion 114 further includes a through hole 114a, which allows the fingers of the operator to pass through, for increasing the utility. In addition, since there are usually more than one targeted vertebrae in the spinal surgery, the hand-held portion 114 can also be used to note the patient name, vertebra location information, and the likes, thereby increasing the safety of the surgery.

FIGS. 1C to 1E are schematic diagrams showing the auxiliary element, k-pin and cannular driller of the guiding assembly for spinal drilling operation according to the embodiment of the present invention. Also reference to FIGS. 1A and 1B, the auxiliary element 12 is mounted on the locating part 113. Preferably, the auxiliary element 12 may further include a hand-held portion 121 for facilitating operations. More preferably, the auxiliary element 12 may further include a through hole 122 to allow the operator to conveniently grab the auxiliary element 12.

The auxiliary element 12 has roughly a pillar shape. The bottom half of the auxiliary element 12 has a diameter and appearance the same as those of the locating part 113. The top half thereof has a slightly larger diameter for limiting the percentage of the locating part 113 mounted therein. With reference to FIGS. 1A to 1E, the k-pin 13 is mounted on the auxiliary element 12, and the cannular driller 14 is mounted on the k-pin 13. The cannular driller 14 includes a holding part 142 and a drilling part 143. In practice, the cannular driller 14 can be any drilling device with a holding part and a threaded drilling head such as, for example but not limited to, a T-shaped driller of this embodiment.

Each of the auxiliary element 12, k-pin 13 and cannular driller 14 is integrally formed or assembled with several parts. Besides, each of the auxiliary element 12, k-pin 13 and cannular driller 14 is metal element, so that each of them can have better durability. The auxiliary element 12 has a hollow longitudinal through hole 123, which has the inner diameter and shape the same as those of the k-pin 13, so that the k-pin 13 can penetrate through the through hole 123. The cannular driller 14 also has a hollow part 141 for receiving the k-pin 13, so that the cannular driller 14 can be disposed in the locating part 113 after the hollow part 141 is mounted on the k-pin 13. Accordingly, the cannular driller 14 is guided to the targeted drilling location.

Furthermore, in order to prevent the repulsion issue caused by remaining resin particles when other elements directly contact with the locating part 113, a stainless metal ring 113b is configured at the inner edge of the through hole 113a of the locating part 113. The metal ring 113b is force fitted with the locating part 113, and the contact surface therebetween has a draft pattern design for facilitating the desired mounting and connection.

The guiding assembly for spinal drilling operation can be fixed at cervical vertebrae, thoracic vertebrae, or lumbar vertebrae so as to assist the spinal drilling operation. When the guiding assembly is applied to the thoracic vertebrae or to the more difficult cervical or lumbar vertebrae, the opposite inner sides of the fixing portion 111 c have surface curvatures fitting the appearance of two outer sides of the cervical vertebrae, thoracic vertebrae, or lumbar vertebrae. Thus, the fixing portion 111c can totally attach to the laminas of the cervical vertebrae, thoracic vertebrae, or lumbar vertebrae without interfering the joint capsule and ligamentum flavum, thereby improving the surgery safety.

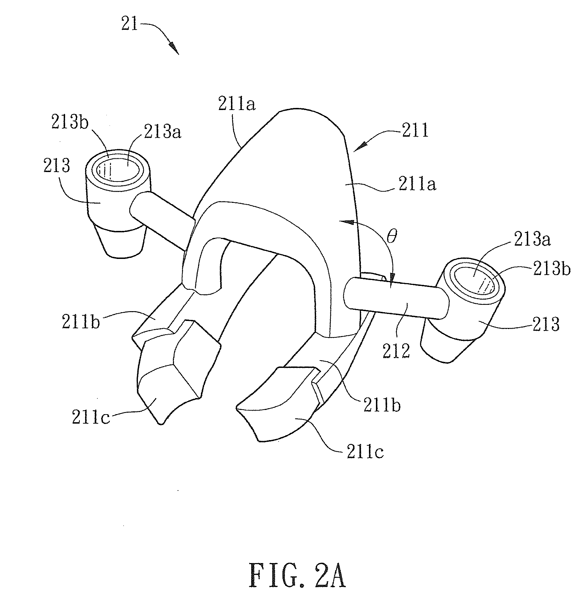

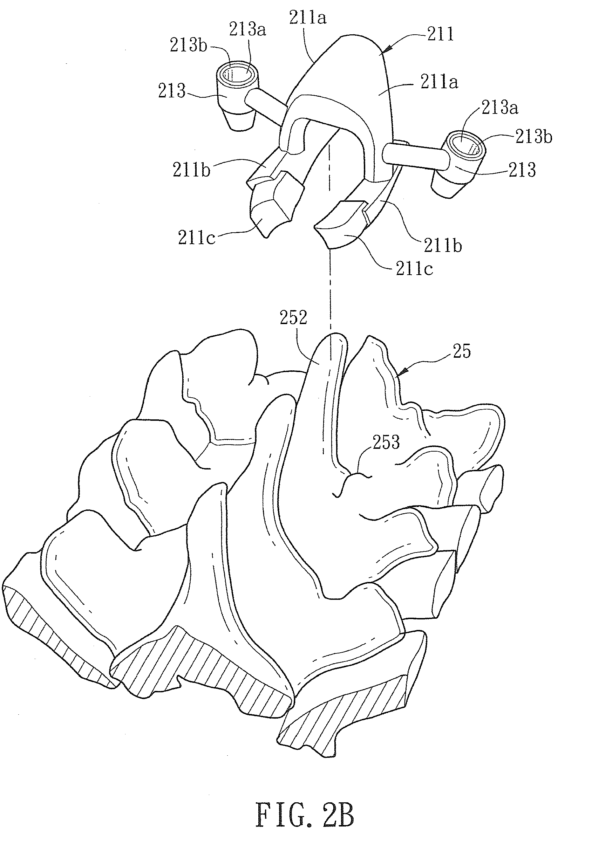

FIG. 2A is a schematic diagram showing another guiding element 21 of the guiding assembly for spinal drilling. The structure and composition of the guiding element 21 is mostly the same as those of the previously mentioned guiding element 11, except for that the main body 211 of the guiding element 21 has two stand portions 211 a, and the guiding element 21 has two contact portions 211b for connecting with two stand portions 211a. In practice, the configuration of two stand portions 211a allows the main body 211 to stand across the spinous process of the vertebra (see FIG. 2B), and the fixing portions 211c connected to the bottoms of the contact portions 211b may contact with the spinous process 252. To be noted, the present embodiment provides the fixing portion 211 c similar to a clip or forceps structure for enhancing the fixing strength. Besides, since the lamina 253 has a smooth and tilting forwardly area, which easily causes sliding during the operation, the contact portions 221b of the guiding element 21 can contact against one side of the vertebra 25 having the curvature corresponding to the surface of the spinous process 252. This feature can prevent the guiding element 25 from moving forwardly and thus enhance the stability of the drilling/reaming operation.

The number of the stand portions and the locating parts are not limited. Of course, if the number of the stand portions increases, the guiding element can be disposed at more restricted position. If the number of the locating parts increases, the guiding element can be located at more possible positions so as to increase the application flexibility.

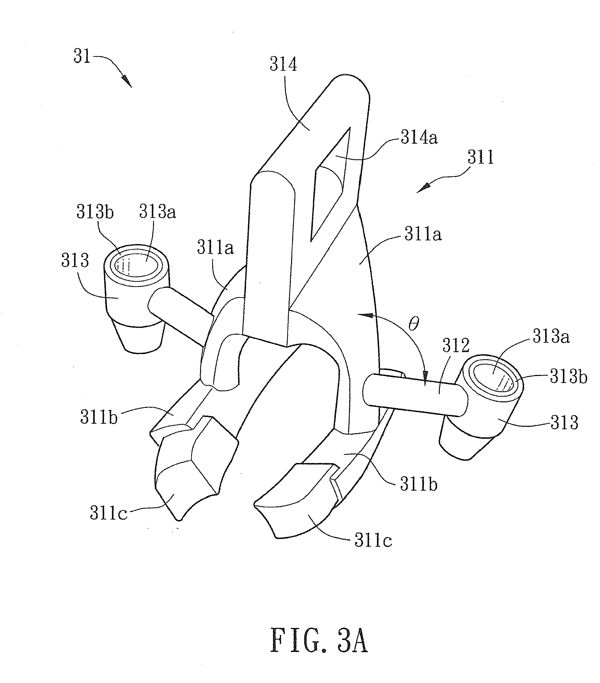

FIG. 3A is a schematic diagram showing another guiding element 31 of the guiding assembly for spinal drilling operation according to the present invention. Referring to FIG. 3A, a hand-held portion 314 connects to the stand portion 311a of the main body 311 of the guiding element 31, so that the operator can easily hold it. Similarly, the space of the hand-held portion 314 can be provided to note some information for identification in surgery. Besides, the hand-held portion 314 may further include a through hole 314a, so that the operator can easily grab the hand-held portion 314 with the through hole 314a to increase the stability.

FIG. 3B is a schematic diagram showing another guiding element 31, not claimed, as well as other elements of the guiding assembly for spinal drilling operation shown in FIGS. 1C to 1E. To be noted, the center line only shows the relative positions of the elements and is not to limit the present invention. Referring to FIG. 3B, the through hole 313a of the guiding element 31 is force fitted with a metal ring 313b and is disposed on the spinous process of the vertebra. Then, the auxiliary element 12 is mounted to the through hole 313a of the locating part 313, followed by mounting the k-pin 13 through the longitudinal through hole 123 of the auxiliary element 12. After that, the k-pin 13 is inserted into the vertebra along the direction of the longitudinal through hole 123. After the direction and angle of the k-pin 13 is fixed, the x-ray is taken to confirm the direction of the k-pin 13. The auxiliary element 12 is stably removed along the axial direction of the k-pin 13, and the hollow portion 141 of the cannular driller 14 is mounted on the k-pin 13. Thus, the drilling part 143 of the cannular driller 14 can pass through the locating part 313 along the axial direction of the k-pin 13 and then be guided to the targeted drilling position. Finally, the operator can manually rotate the cannular driller 14 to expand the hole for implanting.

FIG. 4 is a flow chart of a method, not claimed, for spinal drilling operation. Referring to FIG. 4, the method for spinal drilling operation includes the following steps of: disposing a guiding element on a vertebra, wherein the guiding element includes a main body having at least one stand portion and at least one contact portion connecting with the stand portion, at least one connection part, and at least one locating part connecting to the stand portion through the connection part (step S41); mounting an auxiliary element on the locating part (step S42); mounting a k-pin on the auxiliary element (step S43); locating the k-pin and removing the auxiliary element from the locating part (step S44); mounting a cannular driller having a holding part and a drilling part through the k-pin (step S45); and rotating the holding part to drive the drilling part for a reaming process (step S46). To be noted, the structural features and operation details of the above elements are all described in the above embodiments, so the descriptions thereof will be omitted.

Since the locating part of the guiding element can indicate the calculated drilling position, such as the pedicle, the guiding element of the invention can provide more precise drilling positioning. Moreover, the present invention further uses the auxiliary element and the k-pin to minimize the error range of the center of the pedicle. This can sufficiently improve the conventional error of guiding element positioning during the drilling/reaming operation.

In summary, the guiding assembly for spinal drilling operation of the present invention is composed of several elements, including the guiding element, auxiliary element, k-pin and cannular driller, so that the stability and precision of the reaming procedure during the spinal drilling operation can be enhanced.

Moreover, the specific functions of the above elements can help the spinal pedicle drilling operation. Regarding to the guiding element, since it has simple structure and is suitable for customization, the demand of the patient can be satisfied. Besides, the guiding element has a portion fitting the vertebra, so that the connection between the guiding element and the vertebra can be further improved. In addition, the auxiliary element and the cannular driller are configured cooperating with the locating part. The auxiliary element can assist the location of the k-pin and prevent the non-stability in the conventional hand-hold procedure. The cannular driller can perform the reaming process through the locating part, thereby increasing the precision and decreasing the safety of spinal drilling operation.

Compared with the conventional art, the present invention still remains the conventional advantages of customizable and easy operation, and can further provide higher location stability and precision due to the structural property of the guiding element. Furthermore, either the method, not claimed, of the guiding assembly for spinal drilling operation utilizes an auxiliary element for facilitating and adjusting the penetrating position of the k-pin. This configuration can precisely match the hole drilled by the cannular driller with the targeted pedicle, so that the medical staffs can precisely drill into the center of the pedicle of the targeted vertebra.

Preferably, the guiding element of the present invention can have customized design based on the surface angle of the targeted vertebra (e.g. cervical, thoracic or lumber vertebra) of the patient. Accordingly, when the fixing portion is pressed so as to push the guiding element, the guiding element can be still fixed on the targeted vertebra firmly, thereby increasing the precision and safety of the spinal drilling operation.

Although the invention has been described with reference to specific embodiments, this description is not meant to be construed in a limiting sense. Various modifications of the disclosed embodiments, as well as alternative embodiments, will be apparent to persons skilled in the art. It is, therefore, contemplated that the appended claims will cover all modifications that fall within the true scope of the invention.

## Claims

1. A guiding assembly for spinal drilling operation, which is disposed across a spinous process (252) of vertebra, and contacts against a surface of lamina (253) of the vertebra, comprising:
a guiding element (31) configured for disposing on the vertebra, wherein the guiding element comprises:
a main body (311) comprising two stand portions (311a),
two connection parts (312),
two locating parts (313) connecting to said two stand portions (311a) of the main body through the connection parts (312), respectively; and
two fixing portions (311c);
**characterized in that** the guiding assembly further comprising:
an auxiliary element (12) mounted on one of the locating parts;
a k-pin (13) mounted on the auxiliary element; and
a cannular driller (14) mounted on the k-pin and having a holding part (142) and a drilling part (143),
wherein the main body (311) further comprises two contact portions (311b) and a hand-held portion (314), wherein the contact portions (311b) are connected with the stand portions (311 a), respectively, the contact portions (311b) are disposed along the vertebra, the stand portions extends along a first direction, the contact portion (311b) is protruded from the stand portion (311a) along a second direction, and the first direction is different from the second direction, the fixing portions (311c) are connected to the contact portions (311b), respectively, and the fixing portions (311 c) attach to the laminas of the vertebra;
wherein each of the fixing portions (311c) has a hook shape,
wherein the stand portions (311a) are configured to stand across the spinous process of the vertebra, and hand-held portion (314) is connected to the stand portions (311 a),
wherein the connection parts (312) are direct physically connected to the stand portions (311a), respectively, and the connection parts (312) are connected to the contact portion (311b) through the stand portions (311a), respectively.

2. The guiding assembly of claim 1, wherein one side of each of the contact portions (311b) in contact against the vertebra has a curvature corresponding to the surface of the lamina (253).

3. The guiding assembly of claim 1, wherein the auxiliary element has a through hole along a longitudinal direction, and the k-pin passes through the through hole in the step of mounting the k-pin on the auxiliary element.

4. The guiding assembly of claim 1, wherein one side of each of the fixing portions (311c) in contact against the vertebra has a curvature corresponding to the surface edge of the lamina (253).

## Patentansprüche

1. Führungseinrichtung für eine Bohroperation an der Wirbelsäule, die über einem Wirbelsäulen-Knochenansatz (252) des Vertebras / Wirbelbogens angeordnet ist, und mit einer Oberfläche der Bogenlamina (253) in Kontakt steht, umfassend:
ein Führungs-Element (31), das zum Anordnen auf der Vertebra ausgebildet ist, wobei das Führungs-Element umfasst:
einen Hauptkörper (311), der zwei Ständer-Bereiche (311a), zwei Verbindungs-Teile (312),
zwei Anordnungs-Teile (313), die jeweils mit den zwei Ständer-Bereichen (311a) des Hauptkörpers über die Verbindungs-Teile (312) verbunden sind; und
zwei Fixier-Teile (311c);
**dadurch gekennzeichnet, dass** die Führungseinrichtung des Weiteren umfasst:
ein Unterstützungs-Element (12), das auf einem Anordnungs-Teile (313) angebracht ist;
einen k-Stift (13), der auf dem Unterstützungs-Element (12) angebracht ist; und
ein röhrenförmiger Bohrer (14), der auf dem k-Stift (13) angebracht ist und einen Halte-Teil (142) und einen Bohr-Teil (143) aufweist,
wobei der Hauptkörper (311) des Weiteren zwei Kontakt-Teile (311b) und einen Handgriff (314) umfasst, wobei die Kontakt-Teile (311b) jeweils mit den Ständer-Bereichen (311a) verbunden sind und wobei die Kontakt-Teile (311b) über die Vertebra hinweg angeordnet sind, und wobei die Ständer-Bereiche sich entlang einer ersten Richtung erstrecken, und wobei sich der Kontakt-Teil (311b) von dem Ständer-Teil (311a) entlang einer zweiten Richtung erstreckt, und wobei die erste Richtung verschieden von der zweiten Richtung ist, und wobei die Fixier-Teile (311c) jeweils mit den Kontakt-Teilen (311b) verbunden sind, und wobei die Fixier-Teile (311c) an die Bogenlamina angebracht sind;
wobei jeder der Fixier-Teile (311c) eine Haken-Form aufweist,
wobei die Ständer-Bereiche (311a) angeordnet sind, um über dem Wirbelsäulen-Knochenansatz des Vertebras zu stehen, und wobei der Handgriff (314) mit den Ständer-Bereichen (311a) verbunden ist, wobei die Verbindungs-Teile jeweils direkt, physikalisch mit den Ständer-Bereichen verbunden sind, und wobei die Verbindungs-Teile jeweils mit den Kontakt-Teilen (311b) über die Ständer-Bereiche verbunden sind.

2. Führungseinrichtung nach Anspruch 1, wobei eine Seite von jedem der Verbindungs-Teile (311b), der in Kontakt mit der Vertebra ist, eine Krümmung aufweist, die der Oberfläche der Bogenlamina (253) entspricht.

3. Führungseinrichtung nach Anspruch 1, wobei das Unterstützungs-Element ein Durchloch in einer longituditionalen Richtung aufweist, und wobei der k-Stift in dem Schritt des Befestigens des k-Stiftes auf dem Hilfs-Element, durch das Durchloch hindurch ragt.

4. Führungseinrichtung nach Anspruch 1, wobei eine Seite von jedem der Fixier-Teile (311c), das in Kontakt mit Vertebra ist, eine Krümmung aufweist, die der Außenkante der Bogenlamina (253) entspricht.

## Revendications

1. Un ensemble de guidage pour une opération de forage de la colonne vertébrale, qui est disposé au travers d'une apophyse épineuse (252) vertébrale, et qui est en contact contre une surface de lame (253) de la vertèbre, comprenant :
un élément de guidage (31) configuré pour être disposé sur la vertèbre, dans lequel l'élément de guidage comprend :
un corps principal (311), comprenant de parties de support (311a),
deux parties de connexion (312),
deux parties de positionnement (313) se raccordant aux dites deux parties de support (311a) du corps principal au travers des parties de connexion (312), respectivement ; et
deux parties de fixation (311c) ;
**caractérisé en ce que** l'ensemble de guidage comporte en outre :
un élément auxiliaire (12) (12) monté sur l'une des parties de localisation;
une broche k (13) montée sur l'élément auxiliaire; et
un foreur de canule (14) monté sur la broche k et ayant une partie de maintien (142) et une partie de forage (143),
dans lequel le corps principal (311) comporte en outre deux parties de contact (311b) et une partie de maintien (314), dans lequel les parties de contacts (311b) sont connectés aux parties de support (311a), respectivement, les parties de contact (311b) sont disposées le long de la vertèbre, les parties de support s'étendant le long d'une première direction, la partie de contact (311b) faisant saillant par rapport à la partie de support (311a) le long d'une seconde direction, et la première direction étant différente de la seconde direction, les parties de fixation (311c) étant connectée aux parties de contact (311b), respectivement, et les parties de fixation (311c) s'attachant aux lames de la vertèbre ;
dans lequel chacune des parties de fixation (311 c) présente une forme de crochet,
dans lequel les parties de support (311a) sont configurées pour se tenir contre l'apophyse épineuse de la vertèbre, et la partie de maintien (3144) est connectée aux parties de support (311a),
dans lequel les parties de connexion sont directement physiquement connectées aux parties de support (311a), respectivement, et les parties de connexion (312) sont connectées à la partie de contact (311 b) au travers les parties de support (311a), respectivement.

2. L'ensemble de guidage de la revendication 1, dans lequel un côté de chacune des parties de contact (311b) en contact avec la vertèbre présente une courbure correspondant à la surface de la lame.

3. L'ensemble de guidage de la revendication 1, dans lequel l'élément auxiliaire comporte un trou traversant le long d'une direction longitudinale, et la broche k passe à travers du trou traversant lors du montant de la broche k sur l'élément auxiliaire.

4. L'ensemble de guidage de la revendication 1, dans lequel un côté de chacune des parties de fixation (311c) en contact contre la vertèbre présente une courbature correspondant au bord de la lame.
